# EUROPEAN PATENT APPLICATION

(11) **EP 3 020 356 A1**
(43) Date of publication of application: **18.05.2016**
(21) Application number: 14822581.6
(22) Date of filing: 17.06.2014
(51) Int. Cl.: A61B 90/00

(54) **SURGICAL ASSISTANCE ROBOT**

(30) Priority: 12.07.2013 JP 2013146056
(71) Applicant: Olympus Corporation, Shibuya-ku Tokyo 151-0072 (JP)
(72) Inventor: YORIMOTO, Ryuichi, Tokyo 151-0072 (JP)
(74) Representative: Gunzelmann, Rainer
(86) International application number: PCT/JP2014/066011
(87) International publication number: WO 2015/005072

(57) **Abstract**

The invention has for its object to provide a surgical robot that makes sure good enough operation even when it is difficult to have an instantaneous grasp of a three-dimensional position relationship between an affected site and a treatment tool or have a full understanding of force interacting between the affected site and the treatment tool. The surgical robot 1 disclosed herein includes an operating assembly, a distal end portion that moves depending on an amount of operation by the operating assembly, a treatment tool located at said distal end potion, a detection sensor located near the distal end portion to detect states of the distal end portion, and an actuation mode changeover portion (Steps S102, S103 and S104) configured to make a change to actuation modes depending on the result of detection by the detection sensor.

## Description

### TECHNICAL FIELD

The present invention relates to a medical surgery support robot (surgical robot) that is used while inserted through the body cavity to apply treatments to various in-vivo tissues.

### BACKGROUND ART

A surgical robot comprising a master manipulator operated by an operator and a slave manipulator for performing treatments on the basis of the operation of the master manipulator has so far been known as a surgery support system.

There is endoscopic mucosal resection now available wherein such a surgical robot is used to insert the slave manipulator through the body cavity to cut off affected sites or lesions on mucosal tissues by means of a treatment tool. Such surgical operation is expected to have a wider range of applications because there is no need for abdominal operation, resulting in limited burdens on patients and some consideration curtailments in the number of days taken until postoperative recuperation and leaving hospital.

For instance, Patent Publication 1 (JP(A) 2006-325744) discloses an endoscopic operation system for performing such operation, which system comprises an endoscope in which the distal end of a treatment tool channel is open along the longitudinal axis direction of an insert portion so that the distal end of the treatment tool inserted into the treatment tool channel can extend along the longitudinal axis direction of the insert portion, wherein the incoming optical axis of an objective optical system located in combination with the opening at the distal end of the insert portion tilts toward the treatment tool channel side.
Patent Publication 1: JP(A) 2006-325744

### OBJECT(S) OF THE INVENTION

Browsable information used for performing treatments in the body cavity with the use of a surgical robot in conventional endoscopic operation systems is only images obtained through an endoscope. Problems with them are that they are poor in operability: it is difficult to have an instantaneous grasp of a three-dimensional position relationship between an affected site and a treatment tool, it is difficult to have a full understanding of force interacting between the affected site and the treatment tool, and so on.

### SUMMARY OF THE INVENTION

To provide a solution to the aforesaid problem, the invention provides a surgical robot, comprising:
an operating assembly,
a distal end portion that moves depending on an amount of operation of the operating assembly,
a treatment tool disposed at the distal end portion,
a detection sensor that is located near the distal end portion to detect a state of the distal end portion, and
an actuation mode changeover portion configured to make a change to actuation modes in response to a result of detection by the detection sensor.

In the surgical robot according to the invention, the detection sensor is a strain sensor.

In the surgical robot according to the invention, the detection sensor is an image sensor.

In the surgical robot according to the invention, the detection sensor is a displacement sensor.

In the surgical robot according to the invention, the actuation mode changeover portion configured to make a change to a relationship between the amount of operation by the operating assembly and the amount of bending of the distal end portion.

In the surgical robot according to the invention, the actuation mode changeover portion configured to make a change to operation by the operating assembly, and a mode of movement of the distal end portion.

In the surgical robot according to the invention, the mode of movement changed by the actuation mode changeover portion includes a first mode of movement that permits for three-dimensional movement, and a second mode of movement that permits for only movement in a plane.

The surgical robot according to the invention further includes a power supply for supplying power to the treatment tool, wherein the actuation mode changeover portion configured to make a change to a power supplied from the power supply to the treatment tool.

### ADVANTAGES OF THE INVENTION

The surgical robot according to the invention includes a detection sensor for detection of states of the distal end portion, and the control process is changed over to a more reliable actuation mode depending on the result of detection by that detection sensor. Thus, even when it is difficult to have an instantaneous grasp of a three-dimensional position relationship between an affected site and a treatment tool or to have a full understanding of force interacting between the affected site and the treatment tool, good enough operation is then achievable.

### BRIEF EXPLANATION OF THE DRAWINGS

Fig. 1 is illustrative in schematic of the construction of the surgical robot 1 according to the first embodiment of the invention.
Fig. 2 is illustrative in schematic of the actuation of the surgical robot 1 according to the first embodiment of the invention.
Fig. 3 is a block diagram for the surgical robot 1 according to the first embodiment of the invention.
Fig. 4 is illustrative in schematic and perspective of the construction of the distal end portion of the surgical robot 1 according to the first embodiment of the invention.
Fig. 5 is a control flowchart for the surgical robot 1 according to the first embodiment of the invention.
Fig. 6 is illustrative of an exemplary actuation of the surgical robot 1 according to the first embodiment of the invention.
Fig. 7 is indicative of actuating conditions stored in the mode table 76 for the surgical robot 1 according to the first embodiment of the invention.
Fig. 8 is illustrative in schematic and perspective of the construction of the distal end portion of the surgical robot 1 according to the second embodiment of the invention.
Fig. 9 is a control flowchart for the surgical robot 1 according to the second embodiment of the invention.
Fig. 10 is illustrative of an exemplary actuation of the surgical robot 1 according to the second embodiment of the invention.
Fig. 11 is indicative of actuating conditions stored in the mode table 76 for the surgical robot 1 according to the second embodiment of the invention.
Fig. 12 is a block diagram for the surgical robot 1 according to the third embodiment of the invention.
Fig. 13 is a control flowchart for the surgical robot 1 according to the third embodiment of the invention.
Fig. 14 is indicative of actuating conditions stored in the mode table 76 for the surgical robot 1 according to the third embodiment of the invention.

### MODES FOR CARRYING OUT THE INVENTION

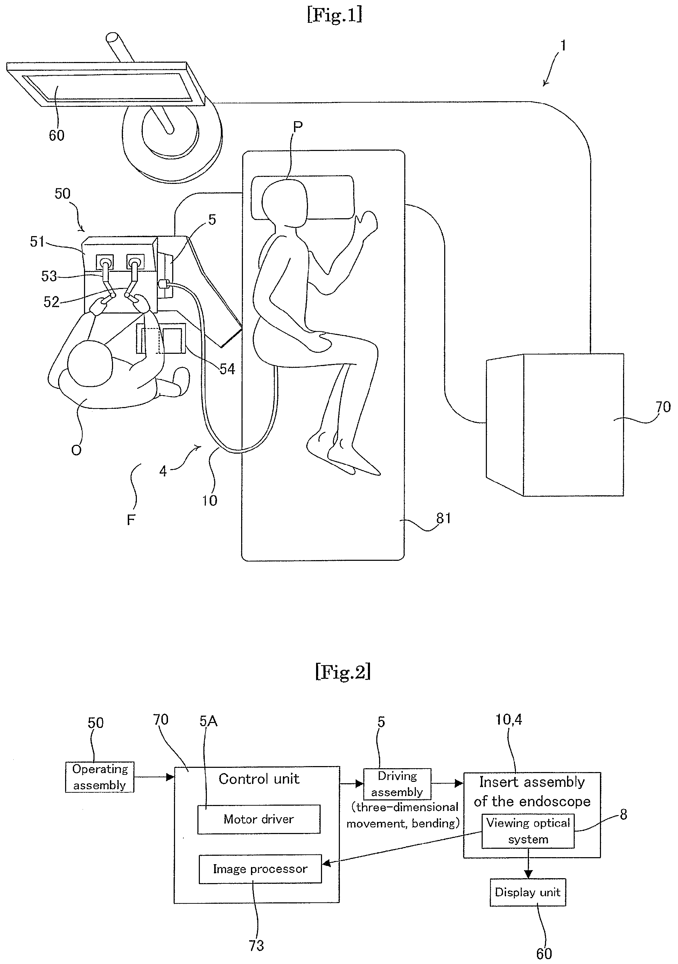

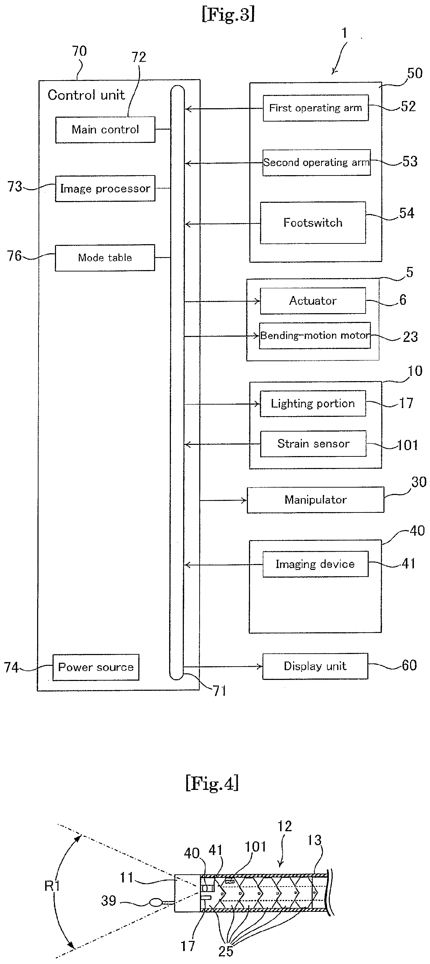

Some embodiments of the invention are now explained with reference to the accompanying drawings wherein Fig. 1 is illustrative in schematic of the construction of the surgical robot 1 according to a specific embodiment of the invention; Fig. 2 is illustrative in schematic of an exemplary actuation of the surgical robot 1 according to a specific embodiment of the invention; Fig. 3 is a block diagram for the surgical robot 1 according to a specific embodiment of the invention; and Fig. 4 is illustrative in schematic and perspective of the distal end portion of the surgical robot 1 according to a specific embodiment of the invention.

The surgical robot 1 according to the first embodiment of the invention, and how to control it, is now explained with reference to the drawings.

As shown in Figs. 1 to 4, the surgical robot 1 according to the embodiment described here is an endoscope system of the master/slave mode comprising an operating assembly 50 operated by an operator O, an endoscope 4 having a flexible insert assembly 10 to be inserted into the body cavity of a patient P, for instance, a limp internal organ such as the large intestine, a driving assembly 5 for performing driving movements such as insertion of the insert assembly 10 of the endoscope 4, bending of the distal end of the insert assembly 10 and twisting of the insert assembly 10 on the proximal end side of the insert assembly 10, a control unit 70 for gaining control of the driving assembly 5, and a display unit 60 for displaying images obtained through the endoscope 4.

As shown in Fig. 1, the operating assembly 50 includes a pair of operating arms 52, 53 attached to an operating mount 51, and a footswitch 54 placed on a floor surface F.

The operating arms 52, 53 are each of a multi-joint structure. The operating arm 52 is provided to operate the bending movement of the bending portion 12 of the insert assembly 10 while the operating arm 53 is provided to operate the three-dimensional movement of the rigid distal-end portion 11.

As shown in Fig. 2, the endoscope 4 includes a viewing optical system 8 for obtaining in-vivo images at the distal end of the insert assembly 10. Images obtained through the viewing optical system 8 are sent out to an image processor 73 disposed within the control unit 70.

As shown in Figs. 1 to 4, the endoscope 4 according to the embodiment described here comprises an insert assembly 10 inserted in the body, a treatment tool 39 and an imaging assembly 40 located at the rigid distal-end portion 11 positioned at the distal end of the insert assembly 10, an operating assembly 50 operated by an operator O such as a surgeon to produce out an operational instruction (instruction), a display unit 60 for displaying images obtained through the imaging assembly 40, and a control unit 70 for gaining control of the insert assembly 10 pursuant to the operational instruction.

The insert assembly 10 is a so-called flexible insert assembly that, as shown in Fig. 4, includes the aforesaid rigid distal-end portion 11, a bending portion 12 that is positioned on a distal end side with respect to the rigid distal-end portion 11 and capable of being bent, and a flexible tubular portion 13 positioned on a distal end side with respect to the bending assembly 12.

The rigid portion 11 of the distal end is formed of a transparent member transparent to light emitted out of a lighting portion 17 such as an LED. The rigid portion 11 of the distal end is less bendable than the bending assembly 12. This rigid distal-end portion 11 is provided with a treatment tool 39 such as an electrical knife.

The imaging assembly 40 has a built-in imaging device 41 such as a CCD (see Fig. 3) on its distal end side. The imaging device 41 is capable of acquiring images within a range of visual field R1, and then converting them into signals to be sent out to the control unit 70.

The bending assembly 12 used may have a configuration well known in the art. Although not illustrated, the bending assembly 12 comprises a plurality of joint rings 25 connected together in such a way as to be mutually rotatable and arranged in an axial direction of the insert assembly 10. The distal one of such joint rings is connected with the distal ends of four operating wires (not shown) at an equiangular interval around the axis of the bending assembly 12. The operating wires are each connected at the proximal end to a bending-motion motor 23 that is positioned on the proximal end of the insert assembly 10 (see Fig. 3). The proximal ends of the operating wires are pulled or towed by the bending-motion motor 23 to bend or flex the bending assembly 12 in any desired bow direction.

A joint ring 25 positioned near the rigid distal-end portion 11 is provided with a strain sensor 101 to detect a state of the rigid distal-end portion 11. In the embodiment described here, this strain sensor 101 is used to detect the stress applied on the rigid distal-end portion 11.

As illustrated in Figs. 1 and 3, the operating assembly 50 includes a pair of operating arms 52, 53 mounted on an operating mount 51, a footswitch 54 on a floor surface F, and so on. The operating arms 52, 53 are each of a multi-joint structure. The operating arm 52 is provided to bend the bending assembly 12 of the insert assembly 10.

On the other hand, the operating arm 53 is provided to operate the three-dimensional movement of the rigid distal-end portion 11. The rigid distal-end portion 11 is moved by an actuator 6 forward, vertically or horizontally.

Upon put in operation, the operating arms 52, 53 produce out an operational instruction to the control unit 70. Thus, the operating assembly 50 may produce out an operational instruction to operate the bending assembly 12, manipulator 30 and imaging assembly 40 by way of the control unit 70.

As illustrated in Fig. 1, the display unit 60 is located in a position in opposition to the operator O as the operator grips the operating arms 52, 53. The display unit 60 is connected to the control unit 70.

The driving assembly 5 comprises an actuator 6 for driving the rigid distal-end portion 11 of the insert assembly 10 ahead in the longitudinal direction. As the operator actuates the operating assembly 50 to drive the insert assembly 10 ahead in the longitudinal direction, it causes the actuator 6 to be driven to move the insert assembly 10 forward or backward.

The control unit 70 generates an instruction signal for driving the actuator 6 in the driving assembly 5 on the basis of an operational signal from the operating assembly 10. That is, the control unit 70 is designed to calculate an amount of movement of the rigid distal-end portion 11 by the actuator 6 on the proximal end side of the insert assembly 10 within a certain period of time, sending such an instruction signal as to achieve said amount of movement out to the driving assembly 5.

As shown in Fig. 3, the control unit 70 includes a main control 72 connected to a bus 71, an image processor 73, and a power supply 74. The bus 71 is connected with the bending-motion motor 23 in the insert assembly 10, the imaging device 41 in the imaging assembly 40, the strain sensor 101, the operating arms 52, 53 and footswitch 54 in the operating assembly 50, the display unit 60, and the mode table 76, respectively.

The main control 72 and image processor 73 are each comprised of a logic element, a memory, a control program, and so on. Pursuant to an operational instruction produced out of the operating arm 52 to the bending assembly 12 in the insert assembly 10, the main control 72 drives the bending-motion motor 23 to pull the proper operating wire thereby bending the bending assembly 12. Pursuant to an operational instruction produced out of the operating arm 53 to the actuator 6, the main control 72 drives the actuator 6 to move the rigid distal-end portion 11 of the insert assembly 10.

In the mode table 76 or a memory for the main control 72, the actuating conditions used in a common or ordinary mode, and the actuating conditions used in a more reliable mode are stored. The main control 72 determines the control mode of the surgical robot 1 with reference to readings taken from the strain sensor 101 and that mode table 76.

While the control unit 70 includes a motor driver for driving a motor such as the bending-motion motor 23 in the driving assembly 5, as shown in Fig. 2, it is to be understood that the motor driver may be provided in the driving assembly 5 apart from the control unit 70 as an example. In this case, the motor driver comprising a logic element, a memory, a control program, and so on may drive a motor such as the bending-motion motor 23, even when it is not electrically connected to the control unit 70.

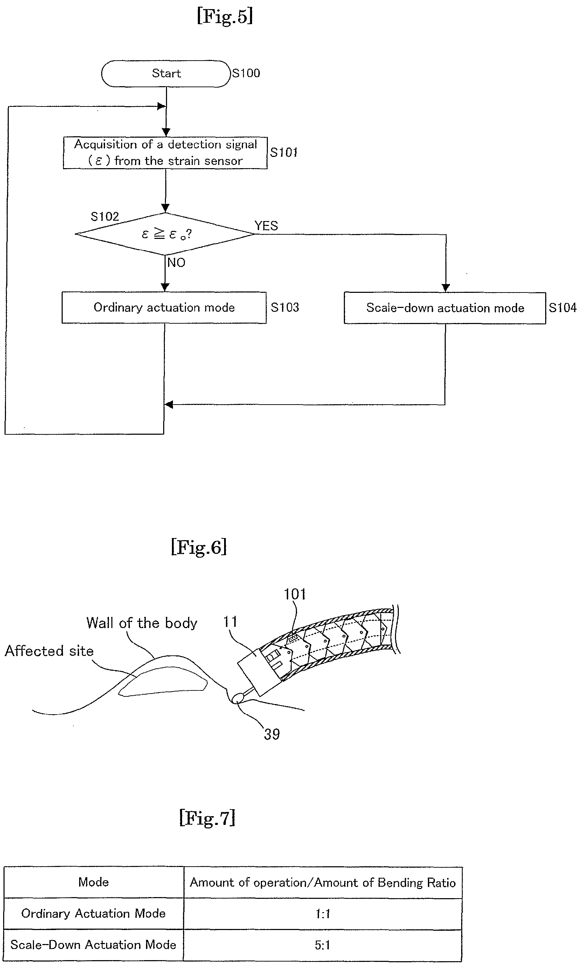

An exemplary control process for the surgical robot 1 according to the invention is now explained. Fig. 5 is a control flowchart for the surgical robot 1 according to the first embodiment of the invention; Fig. 6 is illustrative of an exemplary actuation of the surgical robot 1 according to the first embodiment of the invention; and Fig. 7 is indicative of actuating conditions stored in the mode table 76 for the surgical robot 1 according to the first embodiment of the invention. Note here that the aforesaid flowchart is devoted to a modified algorithm for the actuating mode of the surgical robot 1.

Referring to Fig. 5, the control process gets started in Step S100, and then goes to Step S101 in which a detection signal (ε) is obtained out of the strain sensor 101.

In Step S102, whether or not the detection signal ε is greater than a given value ε₀ is determined. If the result of the determination is YES indicating that the rigid distal-end portion 11 or the treatment tool 39 abuts on an internal organ to apply force to it, the control process then goes to Step S104 in which the surgical robot 1 is put in actuation in a scale-down actuation mode. Fig. 6 shows a state of the rigid distal-end portion 11 or treatment tool 39 in abutment on the internal organ. The ratio between the amount of operation of the first operating arm 52 and the amount of bending of the bending assembly near the rigid distal-end portion 11 in such a scale-down actuation mode is typically defined as shown in the mode table 76 of Fig. 7. That is, the actuation in the scale-down actuation mode is limited to 1/5 in the ordinary actuation mode so that finer actuation and good enough operation are achievable.

On the other hand, if the result of the aforesaid determination is NO indicating that the rigid distal-end portion 11 or treatment tool 39 is not in abutment on the internal organ, the control process then goes to Step S103 in which the surgical robot 1 is put into actuation in the ordinary actuation mode defined in the mode table 76.

As described above, the surgical robot 1 according to the invention includes a detection sensor (strain sensor 101) capable of detecting the state of the distal end portion, and depending on the result of detection by this detection sensor (strain sensor 101), the control process is changed over to a more reliable actuation mode (scale-down actuation mode). Thus, even when it is difficult to have an instantaneous grasp of a three-dimensional position relationship between an affected site and the treatment tool or have a full understanding of force interacting between the affected site and the treatment tool, good enough operation is then achievable.

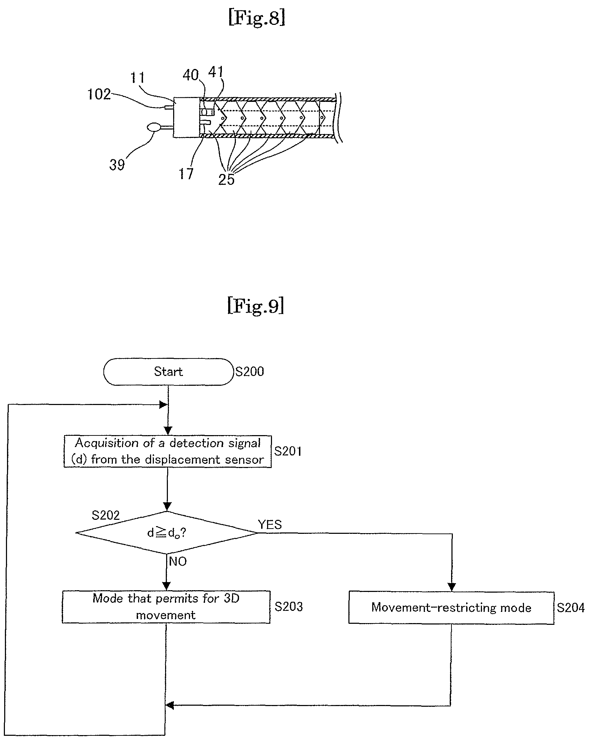

The second embodiment of the invention is now explained. Fig. 8 is illustrative in schematic and perspective of the configuration of the distal end portion of the surgical robot 1 according to the second embodiment of the invention. In the first embodiment of the invention, the strain sensor 101 is used as the detection sensor for detection of a state of the rigid distal-end portion 11 or treatment tool 39, and the scale-down actuation mode is used as the actuation mode more reliable than the ordinary actuation mode. In the second embodiment of the invention, on the other hand, a displacement sensor 102 is used as the detection sensor for detecting a state of the rigid distal-end portion 11 or treatment tool 39, and a movement-restricting mode is used as an actuation mode more reliable than the ordinary actuation mode. The displacement sensor 102 detects a displacement of a pole-like member extending from the rigid distal-end portion 11 to send a detection signal out to the main control 72 by way of the bus 71.

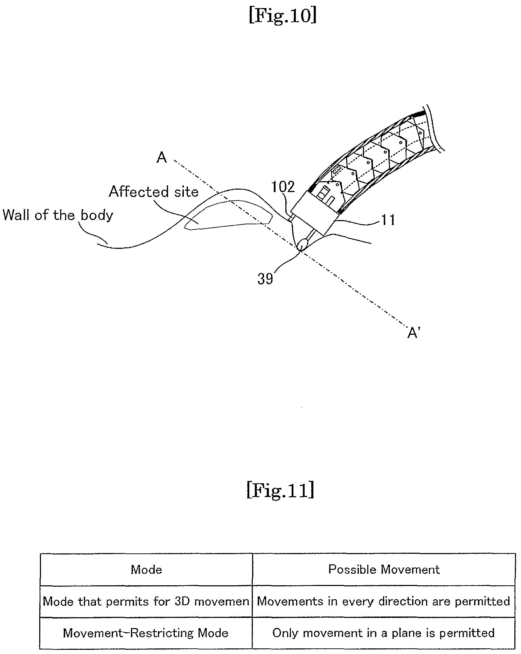

An exemplary control process of the surgical robot 1 according to the second embodiment of the invention is now explained. Fig. 9 is a control flowchart for the surgical robot 1 according to the second embodiment of the invention; Fig. 10 is illustrative of an exemplary actuation of the surgical robot 1 according to the second embodiment of the invention; and Fig. 11 is indicative of actuating conditions stored in the mode table 76 for the surgical robot 1 according to the second embodiment of the invention. Note here that the aforesaid flowchart is devoted to a modified algorithm for the actuation mode of the surgical robot 1.

Referring to Fig. 9, the control process get started in Step S200, and then goes to Step S201 in which there is a detection signal (d) obtained from the displacement sensor 102.

In Step S202, whether or not the detection signal d is greater than a given value do is determined. If the result of the determination is YES indicating that the rigid distal-end portion 11 abuts on an internal organ enough for the pole-like member of the displacement sensor 102 to be forced in the internal organ, the control process then goes to Step S204 in which the surgical robot 1 is actuated in the movement-restricting mode. Fig. 10 shows a state of the rigid portion 11 of the distal end abutting on the internal organ enough for the pole-like member of the displacement sensor 102 to be forced in the internal organ.

In such a movement-restricting mode as described above, only movement of the rigid portion 11 of the distal end in a given plane permits through the operation of the second operating arm 53. Such a mode is typically defined as shown in the mode table 76 of Fig. 11. In such a movement-restricting mode, even when the rigid portion 11 of the distal end is forcibly moved forward by the operation of the second arm 53, the movement of the rigid portion 11 of the distal end is restricted such that it is only movable within a plane shown by A-A' in Fig. 10 (including a vertical direction to the sheet surface). Such an operation mode makes sure movement of the treatment tool 39 on the same plane and, hence, good enough operation.

On the other hand, if that determination is NO indicating that the rigid distal-end portion 11 or treatment tool 39 is not in abutment on the internal organ, the control process then goes to Step S203 (a mode that permits for three-dimensional movement as defined in the mode table 76) in which the surgical robot 1 is actuated. In this mode that permits for three-dimensional movement, the rigid distal-end portion 11 is movable in every direction within a three-dimensional space.

As described above, the surgical robot 1 described here includes a detection sensor (displacement sensor 102) for detecting a state of the distal end portion, and depending on the result of detection by this sensor (displacement sensor 102), the process is changed over to a more reliable mode (movement-restricting mode). Thus, even when it is difficult to have an instantaneous grasp of a three-dimensional position relationship between an affected site and a treatment tool or to have a full understanding of force interacting between the affected site and the treatment tool, good enough operation is then achievable.

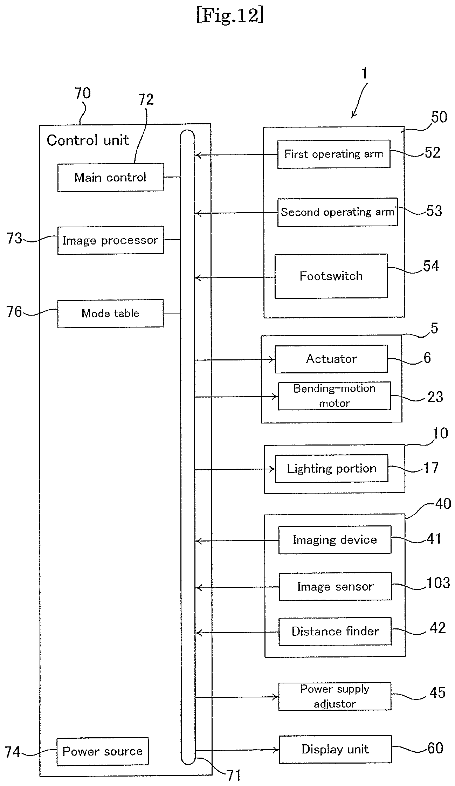

The third embodiment of the invention is now explained. Fig. 12 is a block diagram for the surgical robot 1 according to the third embodiment of the invention. In the first embodiment of the invention, the strain sensor 101 is used as the detection sensor for detecting a state of the rigid portion 11 of the distal end or treatment tool 39, and the scale-down actuation mode is used as a more reliable mode than the ordinary actuation mode. In the third embodiment of the invention, on the other hand, an image sensor 103 and a distance finder 42 are used as the sensor for detecting the state of the rigid portion 11 of the distal end or treatment tool 39, and a reduced power supply mode is used as a more reliable mode than the ordinary actuation mode.

In the embodiment described here, the rigid portion 11 of the distal end is provided with the image sensor 103, and image data acquired there are analyzed by the image processor 73 to identify an affected site. The distance finder 42 is provided at the rigid portion 11 of the distal end to measure a distance from the rigid portion 11 of the distal end to an internal organ or the like and send readings out to the main control 72.

The embodiment described here also includes a power supply adjuster 45 for adjustment of power fed to the treatment tool 39, and the power supply adjuster 45 is controllable in response to a command value from the main control 72.

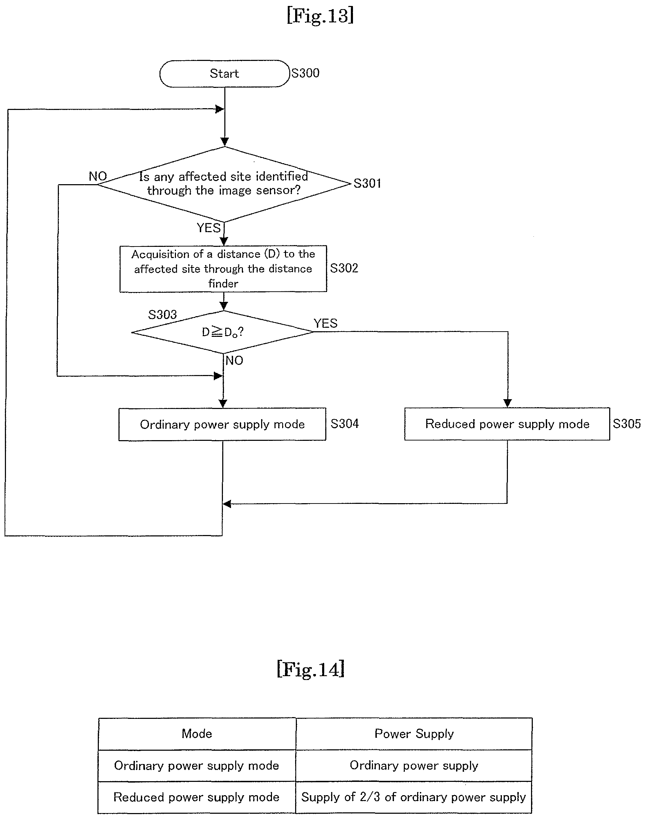

An exemplary control of the surgical robot 1 according to the third embodiment of the invention is now explained. Fig. 13 shows a control flowchart for the surgical robot 1 according to the third embodiment of the invention, and Fig. 14 is indicative of actuating conditions stored in the mode table 76 for the surgical robot 1 according to the third embodiment of the invention. Note here that this flowchart is devoted to a modified algorithm for the actuation mode of the surgical robot 1.

Referring to Fig. 13, the control process gets started in Step S300, and then goes to Step S301 to determine whether or not an affected site is identified on the basis of image data obtained through the image sensor 103. If the determination in Step S301 is NO, the control process then goes to Step S304 in which the actuation mode of the surgical robot 1 is set to an ordinary power supply mode in which the power supply adjuster 45 is actuated to supply ordinary power to the treatment tool 39.

If the determination in Step S301 is YES, on the other hand, the control process then goes to Step S302 to obtain distance data (D) from the distance finder 42.

In Step S303, whether or not the distance data D are greater than a given value Do is determined. If the result of the determination is YES indicating that the rigid portion 11 of the distal end is close to an internal organ, the control process then goes to Step S305 in which the surgical robot 1 is actuated in the reduced power supply mode.

Such a mode is typically defined as shown in the mode table 76 of Fig. 14. In such a reduced power supply mode, the power supply adjuster 45 sets the power supplied to the treatment tool 39 to 2/3 of ordinary power supply. Such an operation mode permits for fine actuation and good enough operation.

If the determination in Step S303 is NO indicating that the rigid portion 11 of the distal end or treatment tool 39 is not proximate to the internal organ, the surgical robot 1 is then actuated in the ordinary power supply mode defined in the mode table 76.

As described above, the surgical robot 1 according to the invention includes the detection sensor (displacement sensor 102) for detecting a state of the distal end portion, and depending on the result of detection by that detection sensor (distance finder 42, image sensor 103), the control process is changed over to a more reliable mode (reduced power supply mode). Thus, even when it is difficult to have an instantaneous grasp of a three-dimensional position relationship between an affected site and a treatment tool or to have a full understanding of force interacting between the affected site and the treatment tool, good enough operation is then achievable.

It is here to be noted that any combinations of the embodiments described herein are all included in the category of the invention, too. In other words, any desired sensor may be used as the detection sensor for detection of states of the distal end portion, and any desired mode may be used as the actuation mode of the surgical robot 1.

Referring to the operating assembly 50 according to each of the embodiments described herein, while the bending assembly 12 and the rigid portion 11 of the distal end are shown as operated by a pair of arms, it is understood that the bending assembly 12 and the rigid portion 11 of the distal end may be operated by one arm. In this case, for instance, the control mode changeover may be done as by the footswitch 54.

It is understood that the treatment tool 39 having a multi-joint structure may also be operated through the control mode changeover done at the operating assembly 50. In this case, one or more treatment tools 39 may be used; for instance, a pair of grip forceps, each as the treatment tool 39, may be operated by a pair of arms. While the structure of the operating assembly 50 has been explained as an arm structure, it is understood that joy ticks may be used instead of arms.

### APPLICABILITY TO THE INDUSTRY

The present invention relates to a medical surgery support robot (surgical robot) that is used while inserted through the body cavity to apply treatments to various in-vivo tissues. So far a surgical robot comprising a master manipulator operated by an operator and a slave manipulator for performing treatments on the basis of the operation of the master manipulator has been known as a surgery support system. There is endoscopic mucosal resection now available wherein such a surgical robot is used to insert the slave manipulator through the body cavity to cut off affected sites on mucosal tissues by means of a treatment tool. Such surgical operation is expected to have a wider range of applications because there is no need for abdominal operation, resulting in limited burdens on patients and some consideration curtailments in the number of days taken until postoperative recuperation and leaving hospital. However, browsable information used for performing treatments in the body cavity with the use of a surgical robot in conventional endoscopic operation systems is only images obtained through an endoscope. Problems with them are that they are poor in operability: it is difficult to have an instantaneous grasp of a three-dimensional position relationship between an affected site and a treatment tool, it is difficult to have a full understanding of force interacting between the affected site and the treatment tool, and so on. By contrast, the surgical robot according to the invention includes a detection sensor for detection of states of the distal end portion, and depending on the result of detection by that detection sensor, the control process is changed over to a more reliable mode. Thus, even when it is difficult to have an instantaneous grasp of a three-dimensional position relationship between an affected site and a treatment tool or to have a full understanding of force interacting between the affected site and the treatment tool, good enough operation is then achievable, contributing more to the industry.

### Explanation of the Reference Numerals

- 1:: Surgical robot
- 4:: Endoscope
- 5:: Driving assembly
- 6:: Actuator
- 8:: Viewing optical system
- 10:: Insert assembly
- 11:: Rigid portion of the distal end
- 12:: Bending assembly
- 13:: Flexible tubular portion
- 17:: Lighting portion
- 23:: Bending-motion motor
- 25:: Joint rings
- 39:: Treatment tool
- 40:: Imaging assembly
- 41:: Imaging device
- 42:: Distance finder
- 45:: Power supply adjuster
- 50:: Operating assembly
- 51:: Operating mount
- 52:: First operating arm
- 53:: Second operating arm
- 54:: Footswitch
- 60:: Display unit
- 70:: Control unit
- 71:: Bus
- 72:: Main control
- 73:: Image processor
- 74:: Power source
- 76:: Mode table
- 81:: Operating table
- 101:: Strain sensor
- 102:: Displacement sensor
- 103:: Image sensor
- O:: Operator
- P:: Patient
- R1:: Range of visual field
- F:: Floor surface

## Claims

1. A surgical robot comprising:
an operating assembly;
a distal end portion that moves depending on an amount of operation of the operating assembly;
a treatment tool disposed at the distal end portion;
a detection sensor that is located near the distal end portion to detect a state of the distal end portion; and
an actuation mode changeover portion configured to make a change to actuation modes in response to a result of detection by the detection sensor.

2. A surgical robot as recited in claim 1, wherein the detection sensor is a strain sensor.

3. A surgical robot as recited in claim 1, wherein the detection sensor is an image sensor.

4. A surgical robot as recited in claim 1, wherein the detection sensor is a displacement sensor.

5. A surgical robot as recited in any one of claims 1 to 4, wherein the actuation mode changeover portion configured to make a change to a relationship between an amount of operation by the operating assembly and an amount of bending of the distal end portion.

6. A surgical robot as recited in any one of claims 1 to 4, wherein the actuation mode changeover portion configured to make a change to operation by the operating assembly, and a mode of movement of the distal end portion.

7. A surgical robot as recited in claim 6, wherein the mode of movement changed by the actuation mode changeover portion includes a first mode of movement that permits for three-dimensional movement, and a second mode of movement that permits for only movement in a plane.

8. A surgical robot as recited in any one of claims 1 to 4, which further includes a power supply for supplying power to the treatment tool, wherein the actuation mode changeover portion configured to make a change to a power supplied from the power supply to the treatment tool.
